# EUROPEAN PATENT SPECIFICATION

(11) **EP 0 438 053 B1**
(45) Date of publication and mention of the grant of the patent: **16.06.1999**
(21) Application number: 91100047.9
(22) Date of filing: 02.01.1991
(51) Int. Cl.: C12N 15/00, C12P 21/08

(54) **Durable engraftment and development of human hematopoietic lineages in normal mammals**
Dauerhafte Verpflanzung und Entwicklung menschlicher hämatopoietischer Zellinien bei normalen Säugetieren
Implantation durable et développement d'une lignée hématopoiétique humaine chez des mammifères normaux

(30) Priority: 15.01.1990 IL 93067; 26.11.1990 US 618303
(43) Date of publication of application: 24.07.1991
(73) Proprietor: YEDA RESEARCH AND DEVELOPMENT COMPANY LIMITED, Rehovot 76100 (IL)
(72) Inventor: Reisner, Yair, Old Jaffa, Tel Aviv (IL)
(74) Representative: VOSSIUS & PARTNER

(56) References cited:
- EP-A- 0 322 240
- SCIENCE. vol. 242, no. 4886, 23 December 1989, LANCASTER, PA US pages 1706-1708; KAMEL-REID, S. & DICK, J.E; "Engraftment of Inmune-Deficient Mice with Human Hematopoietic Stem Cell"
- JOURNAL OF IMMUNOLOGY. vol. 144, no. 9, 01 May 1990, BALTIMORE US pages 3305 - 3311; MURPHY, W.J. et al.: "An absence of T-cells in murine bone marrow allografts leads to an increased susceptibility to rejection by natural killer cells and T cells "

## Description

The present invention relates to non-human chimeric mammals having long-term stable xenogeneic, preferably human, hematopoietic cell lineages, and to methods for their production. In particular, it relates to chimeric mice having long-term stable human B and T cells, obtained by double transplant with bone marrow cells from a human donor and from a severe combined immunodeficient (SCID) mouse, that can be used for the production of human monoclonal antibodies.

Immunodeficient animals are being used as experimental models to study hematopoietic disorders. Xenogeneic bone marrow has been successfully transplanted only between closely related species such as rat and mouse (for review, see: van Bekkum, D.W. and Lowenberg, B. (1985) Bone Marrow Transplantation: Biological Mechanisms and Clinical Practice, Dekker, New York). Recently, attempts have been made to produce a mouse with an immune system of human origin. Such animals can be used as models for the study of human hematopoietic cells and the human immune system. An example is the SCID-hu mouse, obtained by transplanting cells from the human immune system into a natural mutant mouse having an inherited condition called "severe combined immunodeficiency" (SCID). Although engraftment of human cells in these models permitted for the first time the infection of mice with HIV (Namikawa, R., Weilbaecher, K.N., Kaneshima, H., Yee, E.J. and McCune, J.M. (1990) J. Exp. Med. 172, 1055), the survival of human cells was severely limited and the chimeras was therefore transient. Human lymphocytes were not detected in significant numbers in the mouse's peripheral blood beyond 2-3 months post-transplant. Moreover, hematopoietic stem cells from human fetal liver transferred into SCID mice did not engraft or differentiate into T cells unless supplemented with a graft of human fetal thymic epithelium which could be implanted under the kidney capsule (McCune, J.M. et al. (1988) Science 241, 1632, and Namikawa, R., Weilbaecher, K.N., Kaneshima, H., Yee, E.J. and McCune, J.M. (1990) J. Exp. Med. 172, 1055). Another mouse model is based on the transplantation of human hematopoietic stem cells into the genetically immunodeficient Bg/Nu/Xid mouse. Here too, human lymphocytes did not induce graft-versus-host disease ((Kamel-Reid, S. and Dick, J.E. (1988) Science 242, 1706).

Previous clinical studies have suggested that, following transplantation of T cell-depleted bone marrow into human SCID (Reisner, Y. et al. (1983) Blood 61, 341) or leukemia patients (Keever, C.A. et al. (1989) Blood 73, 1340), differentiation of mature T cells from human stem cells is slow, taking from 1-4 months; such T cell differentiation in mice takes about 14-21 days following lethal total body irradiation (TBI). Given these differences in the pace of reconstitution, it can be reasonably hypothesized that in a murine microenvironment, human stem cell differentiation would be inefficient and substantially slower, due to the poor cross-reactivity between murine and human cytokines, as well as between other molecules involved in cellular recognition.

Following heavy suppression of the immune and hematopoietic system, as with lethal TBI, mice die within 2 weeks if bone marrow is not provided. Even if human stem cells could theoretically grow in such mice and reconstitute the mouse's hematopoietic system, their slow rate of differentiation into mature immunocompetent lymphocytes or other leucocytes might prevent them from reaching large enough numbers to protect the mice from infections; furthermore, inadequate replenishing of hematopoietic compartments would not protect the mice from death by hematopoietic failure. On the other hand, sublethal conditioning protocols (e.g., sublethal TBI), which spare substantial numbers of hematopoietic and lymphoid cells, would enable endogenous murine cells to compete effectively with transplanted human cells, and ultimately reject the human graft. Thus, the production of stable human-mouse chimeras by transplantation of human bone marrow into lethally irradiated normal mice has long been considered impossible.

For production of a useful mouse-human chimeric model, conflict between the need for prompt hematopoietic reconstitution on the one hand, and the slow differentiation rate of human T and B cell lineages on the other hand, was resolved in the present invention by using SCID mice as bone marrow donors rather than as bone marrow recipients. This was based on the assumption that the SCID bone marrow cells would promptly reconstitute all hematopoietic lineages except the T and B lymphocytic lineages, which cannot develop from SCID pluripotent stem cells due to the deficiency in rearrangement of antigen receptor genes (Schuler, W. et al. (1986) Cell 46, 963) in SCID mice. To test whether the resultant "empty space" in the thymus and bone marrow could gradually be occupied by, and populated with, normal human T and B cells, the SCID mouse bone marrow was combined with human bone marrow depleted of both T cells and B cells and transplanted into lethally irradiated mice. Depletion of T and B cells in the human marrow was achieved by differential agglutination with soybean agglutinin and subsequent E-rosetting with sheep erythrocytes, resulting in human BM cells termed SBA⁻E⁻ (Reisner, Y. et al. (1981) Lancet ii, 327).

According to the present invention, a stable long-term human-mouse chimerism was achieved by lethally irradiating normal BALB/c mice, transplanting them with human bone marrow cells depleted of T and B cells, followed one day later by T cell- depleted bone marrow cells from SCID mice. As mentioned above, SCID mice were used as bone marrow donors so that all hematopoietic lineages except the T and B cell lineages would be rapidly reconstituted. Phenotypically mature human T and B cells were initially detected in peripheral blood of transplanted mice 2-4 months post-transplant, and continued to be detected more than 9 months post-transplant. Similar lymphoid chimerism in peripheral blood, without apparent graft vs. host disease, was also found when unmanipulated human bone marrow (i.e., not depleted of T and B lymphocytes) was transplanted either intravenously or intraperitoneally.

Thymuses of mice transplanted with T cell-depleted human bone marrow were populated with human lymphocytes predominantly of the CD3⁺CD4⁺CD8⁺ phenotype. Circulating human immunoglobulins (Ig) were detected in 7/20 mice transplanted with human marrow depleted of T and B cells, and in 8/22 recipients of untreated human bone marrow. In 2/4 long-term chimeras, human antibodies specific for the dinitrophenol (DNP) antigen were detected in the serum following primary and secondary immunization with DNP bound to keyhole limpet hemocyanin (DNP-KLH). These results demonstrated that engraftment of human bone marrow resulted in substantial degrees of human lymphoid chimerism can be achieved in normal mice and sustained for prolonged periods. The results also indicate that human T cells can develop in a murine thymus without any additional need for human thymic epithelial cells.

These findings serve as the basis for a more general approach to the production of non-human chimeric mammals having long-term stable human hematopoietic cell lineages, by using other genetically deficient non-human mammal donors or recipients which may allow for different human cell populations to engraft and emerge in suitably treated normal non-human mammals.

The present invention is thus directed to a non-human chimeric mammal M4 having long-term stable xenogeneic hematopoietic cells, comprising a mammal M1, the hematopoietic cells of which have been substantially suppressed or destroyed and replaced by hematopoietic cells from at least two different sources, at least one of the sources being hematopoietic cells originating in a mammal M3 of a species other than that of mammal M1, and at least a second of the sources being hematopoietic cells originating from a mammal M2, of a species other than that of M3, having a genetically-determined hematopoietic deficiency.

The invention also relates to a chimeric non-human mammal, M4, in which the endogenous hematopoietic cells have been replaced by exogenous hematopoietic cells from two different sources, at least one of the substituting hematopoietic cell lineages being of human origin, and the other hematopoietic cell lineage originating from a non-human mammal, M2, having a genetically determined hematopoietic deficiency.

The invention also relates to a method for the production of the above chimeric mammal M4, comprising destroying the immune system and transplanting a combination of suitable sources of pluripotent stem cells of human origin, and originating from a non-human mammal, M2, having a genetically-determined hematopoietic deficiency.

In a preferred embodiment of the present invention, the non-human mammal, M1, is a mouse, and the non-human mammal donor, M2, is a SCID mouse, and the mammal M3 is human.

In another embodiment, the present invention provides a method for producing human monoclonal antibodies which comprises: (a) immunizing a chimeric non-human mammal, M4, according to the present invention, which has long-term stable human B and T cells, with an antigen; (b) fusing suitable antibody-forming cells of the immunized non- human mammal M4 with a suitable fusion partner to produce hybridomas; (c) screening the supernatants of the hybridomas for the presence of the desired antibody; (d) cloning the hybridomas producing the desired antibody and (e) recovering the antibody from the supernatant of these clones.

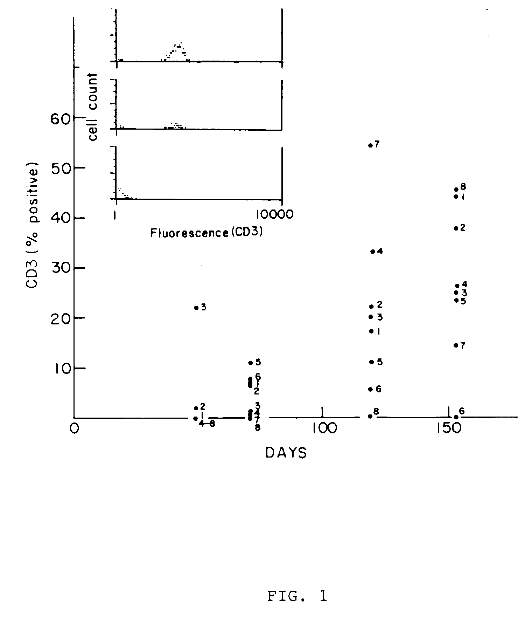

Figure 1 is a graph showing the development of human CD3⁺ cells in the peripheral blood of transplanted mice. Human CD3⁺ cells were analyzed by cytofluorimetry with anti-CD3 (Leu4). Numbers 1-7 which identify the data points refer to surviving mice of the first series of experiments (see Table 1). The inset shows results of a typical experiment carried out on day 49 post-transplant. Staining of normal human peripheral blood lymphocytes (PBL) with anti-CD3 (upper section) is compared to staining of PBL from mouse No. 3 (middle section) and from a control mouse (lower section) transplanted with SCID bone marrow alone. Staining of normal mouse PBL was identical to that found for the control mouse.

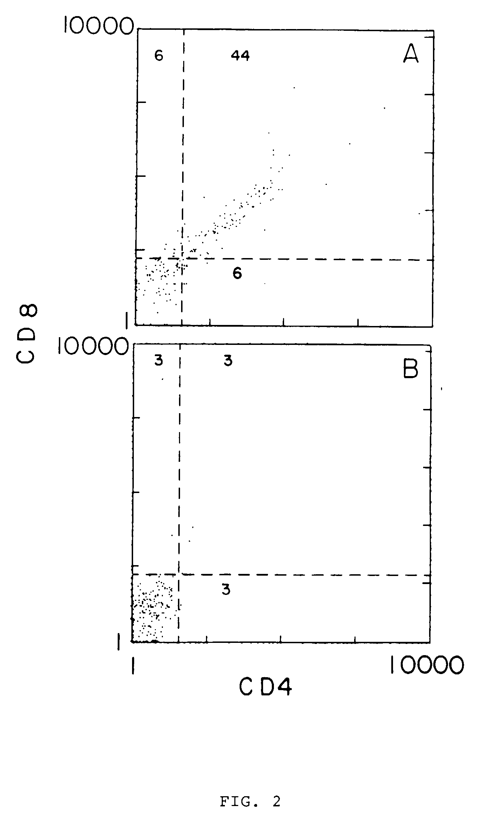

Figure 2 is a graph showing the double staining of thymocytes from a mouse transplanted with human bone marrow, using anti-human CD4 (red) and anti-human CD8 (green). Panel A: The thymus of mouse No. 7 was removed 5 months post-transplant, and the thymocytes were washed twice in phosphate-buffered saline and simultaneously stained with directly-labelled phycoerythrin-conjugated T4 monoclonal antibody and fluorescein isothiocyanate (FITC)- conjugated T8 monoclonal antibody (both from Coulter Immunology, Luton, England). The cells were then washed twice in the buffer and analyzed by cytofluorimetry in a FACS-Scan (Becton-Dickinson, Erembodegem, Belgium). Panel B: Control experiment using normal BALB/c murine thymocytes and the same monoclonal antibodies. The percentages of human CD4⁺CD8⁺, CD4⁺CD8⁻ and CD4⁻CD8⁺ (after subtraction of background staining from control experiment) in the thymus of mouse No. 7 were 41.3%, 3.7% and 3.1%, respectively. (In the thymus of mouse No. 1 these percentages were 42%, 11.7% and 8.2%, respectively.) The percentages of human CD2⁺ cells in the thymuses of mice Nos. 1 and 7 were 35.0% and 47.1%, respectively; percentages of human CD3⁺ cells were 32.0% and 43.0%, respectively.

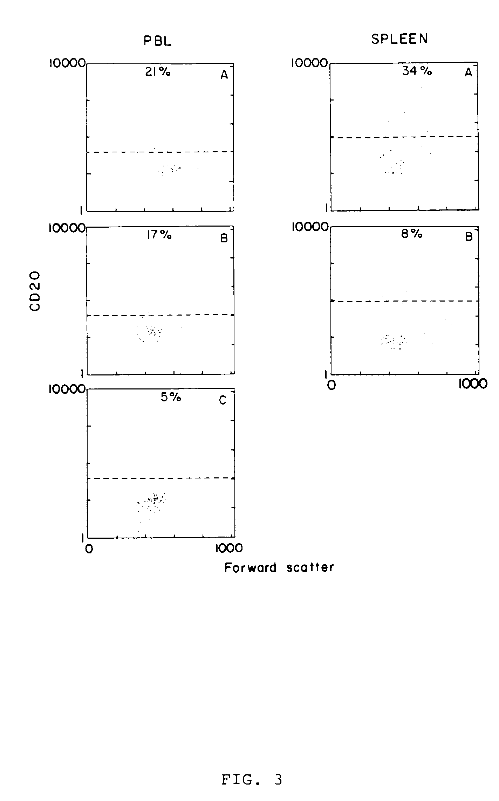

Figure 3 is a graph showing the staining of human B cells in the peripheral blood and spleen of a transplanted mouse. PBL (Left, A) and splenocytes (Right, A) of mouse No. 7 were stained 5 months post-transplant with FITC-conjugated antihuman CD20 monoclonal antibody, analyzed as described in the legend to Fig. 2, and compared to normal human PBL (Left, B), normal mouse PBL (Left, C) and normal mouse splenocytes (Right, B).

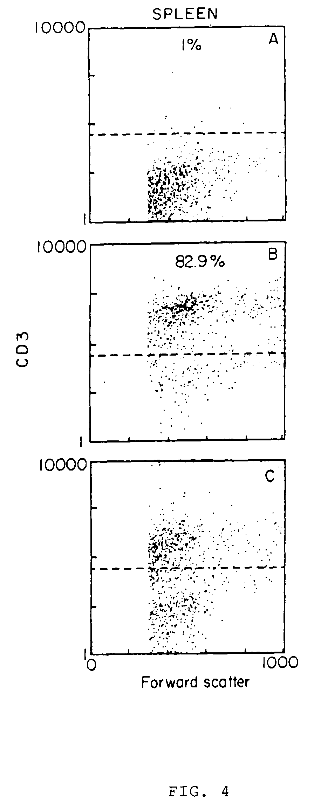

Figure 4 shows the staining of human-mouse chimeric spleen cells with anti-human CD3 after 7 days of culture in IL-2-containing medium. A) Spleen cells from a control mouse transplanted with SCID bone marrow alone. B) Normal human PBL. C) Spleen cells of human-mouse chimera (No. 2 of the first series, 9 months post-transplant) cells (25 x 10³/well) were cultured for 7 days in RPMI medium containing fetal calf serum (10%) and IL-2 (20µg/well).

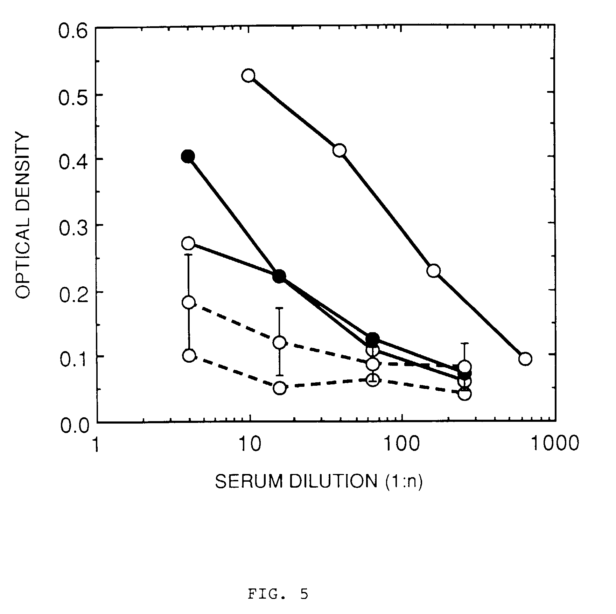

Figure 5 is a graph showing DNP-specific human antibodies in a human-mouse chimera immunized with DNP-KLH. Human-mouse chimera whose PBL were found to be positive for both human T and B lymphocytes, and control mice transplanted only with SCID bone marrow cells, were immunized subcutaneously with 20µg DNP-KLH emulsified in complete Freund's adjuvant. Forty-eight days later, some of the primed mice, as well as some unprimed transplanted mice, were injected subcutaneously with 100µg DNP-KLH emulsified in complete Freund's adjuvant. Mice were bled 6 days later, and anti-DNP antibody was measured by ELISA, as described in the footnotes to Table 1, except that microtiter plates were coated with 100µl/well of DNP-BSA (50µg/ml).

o―o sera of mice Nos. 2 (upper) and 3 (lower) from the first series (challenged twice). ·―· serum of a mouse from the third series (challenged once). o - - - o serum of a control mouse (challenged twice). φ - - - φ average (± standard deviation) of fifteen sera collected from control mice (transplanted with SCID bone marrow alone, and challenged once with KLH-DNP).

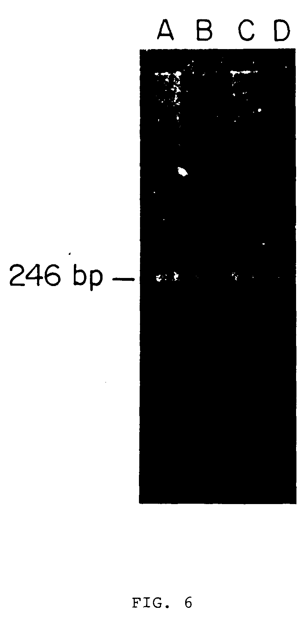

Figure 6 is a gel pattern showing DNA analysis of peripheral blood cells from a human-mouse chimera six months post-transplant. PBL were lysed in distilled water and boiled for 10 min. Debris was sedimented by centrifugation, and the genomic DNA in the supernatant was amplified by the polymerase chain reaction (PCR) procedure for 30 cycles, using DNA polymerase from Thermus aquaticus (Perkin Elmer-Cetus, Emeryville, California, USA) (Saiki, R.K. et al. (1988) Science 239, 487). The reaction mixture, which included 500 ng genomic DNA, 20 pm of the DQB primers GLPDQB1 and GAMPDQB2 (Todd, J.A., Bell, J.I. and McDevitt, H.O. (1987) Nature 329, 599) and 2 U Taq-DNA polymerase, was subjected to repeated cycles of denaturation at 94°C for 30 sec., annealing at 56°C for 30 sec. and extension at 720C for 30 sec. The products were analyzed by 2% agarose gel electrophoresis, where the amplification was regarded as successful only if the control sample from human PBL had a unique 246-bp band (Ronningen, K.S., Iwe, T., Halstensen, T.S., Spurkland, A. and Thorsby, E. (1989) Human Immunology 26, 215). Lane A) normal human PBL; Lane B) an artificial mixture of human and mouse PBL (ratio 1:3); Lane C) PBL from a mouse transplanted with human bone marrow (mouse no. 1); Lane D) PBL from an untreated BALB/c mouse.

The non-human mammal, M1, of the present invention may be any mammal, such as a horse, sheep, or rodent, such as, for example, rabbit, hamster, guinea pig, rat, mouse, etc. The mammal donor M2 having a genetically determined hematopoietic deficiency may be of the M1 species or of a different mammalian species. Preferably, M1 and M2 are of the same species.

The mammal M3 is of a species other than M1, and is preferably human.

The type of human hematopoietic cell lineage expressed in the chimeric mammal M4 will depend on the nature of genetically-determined hematopoietic deficiency of the mammal M2, since transplanted pluripotent stem cells of human origin and originating from mammal M2 will reconstitute the hematopoietic cell lineages of the mammal M1, and only the "empty space" in that reconstituted population resulting from the hematopoietic deficiency of M2 and the inability of M2 spleen cells to generate that lineage will be replaced by cells of the corresponding human hematopoietic lineage.

Thus, in a preferred embodiment of the present invention, a hematopoietically compromised mouse is transplanted with human bone marrow cells and SCID mouse bone marrow cells, leading to production of a mouse having human B and T cells, which are the only lineages which cannot develop from the SCID mouse pluripotent stem cells.

Other human hematopoietic cell lineages such as the erythroid, myeloid, granulocyte, macrophage and megakaryocytoid lineages may be reconstituted in the mammal M1 according to the present invention, yielding different types of chimeras.

According to the invention, any non-human mammal with a genetically determined hematopoietic deficiency may be used as the donor, M2. Useful mouse strains with genetically-determined hematopoietic deficiencies include the immune-deficient Bg/Nu/Xid mouse, and the erythroid-deficient W/W^{v} mouse. Other mouse and mammalian strains will certainly be identified or created by researchers, and will be suitable for use according to the present invention.

A method by which the stable non-human chimeric mammal, M4, may be produced according to the present invention comprises the following steps:
(i) treating a non-human mammal M1 so as to essentially destroy its immune system;
(ii) transplanting the treated mammal M1 with normal human bone marrow cells or any other suitable source of pluripotent stem cells or cells of a particular hematopoietic lineage from a mammal M3, preferably human; and
(iii) additionally transplanting bone marrow cells or any other suitable source of pluripotent stem cells from a non-human mammal donor M2, preferably of the same species as M1, and having a genetically-determined hematopoietic deficiency,
thereby obtaining the chimeric non-human mammal M4 bearing hematopoietic cell lineages derived from mammal donor M2 and one or more M3-derived (e.g. human) hematopoietic cell lineages corresponding to the deficient hematopoietic lineage of said mammal M2.

Although the mammal M3 contributing the hematopoietic cells which are to survive for a long period of time in the chimeric mammal M4 is preferably human, other mammals such as bovine or equine species may be used. Thus, for example, a chimeric mouse having long-term stable bovine T and/or B cells may be produced according to the present invention.

The treatment in step (i) for suppression or destruction of the immune system of mammal M1 may include irradiation (e.g., gamma irradiation), chemotherapy, or a combination of both, or cytoreduction with monoclonal antibodies directed against hematopoietic cells, provided that the treatment allows successful repopulation with pluripotent stem cells from mammal M2.

It is known from previous studies of murine bone marrow transplantation (for review, see: van Bekkum, D.W. and Lowenberg, B. (1985) Bone Marrow Transplantation: Biological Mechanisms and Clinical Practice, Dekker, New York) that, following lethal doses of total body irradiation, some host hematopoietic cells survive the conditioning protocols. Other hematopoietic cells can be generated by surviving early progenitor cells, which have retained their ability to differentiate and proliferate despite the massive doses of radium or chemotherapy. The persistence of host type hematopoietic cells in such chimeras depends not only on the method of conditioning used, but also on the state of the bone marrow used for transplantation. Thus, host-type lymphocytes or other hematopoietic cells will occasionally be found in the human-normal mouse chimeras of the present invention. For this reason, great care must be taken to characterize the chimera's cells for the human phenotypes as well as testing the human origin of antibodies produced by the chimeras.

The source of human pluripotent stem cells may be (a) bone marrow which is either untreated, or T cell-depleted; (b) B and T cell-depleted mature blood cells such as peripheral blood leukocytes; (c) cord blood or fetal tissue (e.g., fetal liver, fetal thymus, fetal bone marrow or fetal lymph nodes). The preferred source of pluripotent stem cells from mammal M2 is bone marrow, either untreated or T cell-depleted, but other suitable sources may be used.

The chimeric mammal M4 of the present invention may be used as an experimental model for the study of a variety of human or veterinary diseases, as well as for other purposes. Thus, chimeric mammals with engrafted human T and B cells may be used for: 1) production of human monoclonal antibodies against antigens such as those associated with pathogens or pathological cells; 2) the induction of tolerance towards human leukemic cells, in mice transplanted with bone marrow from leukemia patients and then infused with the patient's leukemic cells; 3) production of human T cells lines, lymphokine-activated killer (LAK) cells or cytotoxic T lymphocytes (CTLs) to treat human malignancies; and 4) study of human T and B cell ontogeny, which is presently limited largely to in vitro systems.

The results described herein indicate that long-term engraftment of human T cells and B cells can be achieved in lethally irradiated normal mice, following transplantation of T and B cell-depleted bone marrow, without the additional need for a human fetal thymic epithelial graft. Engrafted human T and B lymphocytes appear to be capable of developing responses to mitogenic and antigenic stimuli. The protection from hematopoietic death afforded by the transplant of murine SCID bone marrow allows for the slow generation of human T and B cells in transplanted mice, reasonably resembling the pace of reconstitution in human leukemia patients receiving marrow allografts. Moreover, the presence of immature CD4⁺CD8⁺ (double-positive) human thymocytes in the murine thymus, as described herein, suggests the possibility that differentiation of human bone marrow stem cells into T cells takes place in the mouse's thymic microenvironment.

The ability to produce long-term, vigorous chimeras between human cells and normal mice according to the present invention, contrasts with the much more limited achievements of prior art, in which human bone marrow transplanted into genetically-determined immune-deficient mice did not persist for more than 2-3 months (J.M. McCune et al. (1988) Science 241, 1632, and S. Kamel-Reid and J.E. Dick, Science (1988) 242, 1706.

By the term "long-term stable," as used herein in connection with the cells of the foreign hematopoietic lineage surviving in the chimeric mammal M4, is intended hematopoietic cells, including precursors and differentiated cells, which survive longer than three months. Preferably, such cells are stable and persist in the chimera beyond six months. Even more preferably, such cells persist beyond 1 year, ideally for the lifespan of the chimeric animal.

It was previously shown that human-SCID mouse chimeras could be infected with HIV (R. Namikawa, et al. (1988) Science 242, 1684; D.E. Mosier, et al. (1990) J. Cell. Biochem. Suppl. 14A; 345). The long-term human-normal mouse chimeras of the present invention will provide new and improved murine models for AIDS research. One major advantage over the prior art human-SCID mouse model is based on the fact that SCID mice are generally unhealthy and weak compared to normal BALB/c mice, which easily tolerate bone marrow transplantation. This disadvantage of the prior art human-SCID mouse model is overcome by the present invention, as exemplified by the prolonged survival of the human-normal mouse chimeras of the present invention, even when kept in a non-sterile environment both prior to and following bone marrow transplantation. A further advantage of the present invention is the abundance of human T cells, the target for HIV infection, in these mice, and their prolonged survival.

One embodiment of the present invention, the preparation of human monoclonal antibodies, comprises immunizing the non-human chimeric mammal M4 having human B and T cells with an antigen, removing antibody-forming cells from the spleen or any other appropriate site from the immunized mammal, fusing the suspended cells with a suitable fusion partner to produce hybridomas, appropriately diluting and culturing the cells in separate containers, screening the supernatant in each container containing a hybridoma for the presence of antibody, and selecting and cloning the hybridoma for the presence of antibody, and selecting and cloning the hybridomas producing the desired antibody. These procedures are performed by well-known techniques, described in the literature.

In order to produce the desired monoclonal antibody, the hybridoma cells are either cultured in vitro in a suitable medium, and the desired monoclonal antibody is recovered from the supernatant, or the hybridoma cells are injected intraperitoneally into mice, and the antibodies harvested from the malignant ascites or serum of these mice.

The antigen may be any antigen, e.g., a protein, a cell, etc. The hybridoma fusion partner may be of human origin, or from non-human mammal origin. In a preferred embodiment, it is a mouse myeloma cell.

The invention will now be illustrated by the following examples, herein presented in a non-limiting manner.

### EXAMPLE

### Engraftment of human T and B lymphocytes into lethally irradiated BALB/c mice

(a) Eight to 12 week-old female BALB/c mice (obtained from Olac Farms, Bicester, England) were exposed to a single dose of 10 Gy TBI from a Gammabeam 150-A ⁶⁰Co source (produced by the Atomic Energy of Canada, Kanata, Ontario) with a focal skin distance (F.S.D.) of 75 cm, at a 0.7 Gy/min dose rate. One day later, 10 x 10⁶ T cell- depleted human bone marrow cells [prepared by differential agglutination with soybean agglutinin, followed by E-rosetting with sheep red blood cells, as described by Reisner et al. (Reisner, Y. et al. (1981) Lancet ii, 327)] were transplanted i.v. into each mouse. Twenty-four hours later, T cell-depleted bone marrow from 8- to 12 week-old male SCID mice obtained from the Weizmann Institute Animal Breeding Center) was prepared according to Reisner et al. (Reisner, Y., Itzicovitch, L., Meshorer, A. and Sharon, N. (1978) Proc. Natl. Acad. Sci. USA 75, 2933), with minor modifications (Schwartz, E., Lapidot, T., Gozes, D., Singer, T.S. and Reisner, Y. (1987) J. Immunol. 138, 460), and 2 x 10⁶ cells were transplanted. Mice were bled from the retro-orbital vein, using heparin-coated glass capillaries, at different time intervals, and mononuclear cells were purified by Ficoll-Hypaque fractionation. The presence of human T cells was detected by directly-labeled monoclonal antibodies. These included T3 (anti- CD3), T4 (anti-CD4), T8 (anti-CD8), and T11 (anti-CD2) (all from Coulter Immunology, Luton, England), and Leu4 (anti-CD3) (Becton- Dickinson, Mountain View, California, USA). Human B cells were detected by directly-labelled B1 (anti-CD20) and B4 (anti-CD19) (Coulter Immunology). All these monoclonal antibodies were found to be non-cross-reactive with normal mouse PBL, as can be seen in Figures 1, 2 and 3. Positive mice were scored when more than 2% of their PBL were stained by the appropriate monoclonal antibody (after subtraction of background staining on PBL of normal BALB/c mice). The 2% cutoff was established based on preliminary experiments in which the appropriate antibodies were tested on PBL from a) normal BALB/c mice, b) BALB/c mice transplanted with 2 x 10⁶ T cell-depleted bone marrow cells from SCID donors, and c) humans. It was found that after subtraction of background staining found on cells from group a, the use of a 2% cutoff effectively avoided "false positives" in group b.
(b) Total human Ig content in the serum of transplanted mice was evaluated 3 to 8 months post-transplant by using a double-antibody ELISA. Microtiter plates were coated with 100µl of affinity-purified goat anti-human Fab' antibodies (50µg/ml) (BioMakor, Nes Ziona, Israel). Non-specific binding of protein was eliminated by incubation in a 7% solution of bovine serum albumin (BSA) in PBS for 2 hours. Diluted (1:1) serum from transplanted mice (50µl) was then applied to each well for 16 hours at 4°C. The wells were washed and incubated for 2 hours at 37°C with peroxidase-goat anti-human Ig (IgG, IgM, IgH) (Zemed Laboratory, California), which was incubated with the same volume of normal mouse serum before its final dilutions (to avoid cross-reactivity with Mouse Ig). Following additional washes, substrate solution [2,2- azinodi-(3-ethylbenzthiazoline sulfonic acid) diammonium salt (ATBS)] (Sigma, USA) was applied. The optical density at 620nm was determined by a Titertek Multiscan-ML (Flow Laboratories) ELISA reader. Total Ig concentrations were calculated by reference to a standard curve with purified human Ig. Readings corresponding to more than 20 ng/ml were recorded as positive. This threshold was based on control experiments in which sera of mice transplanted with SCID bone marrow alone was negative. Human IgG and IgM were determined in some mice (see text) as described above, using as second antibodies peroxidase-horse anti-human (IgG)Fc (BioMakor) and peroxidase-goat anti-human IgM(µ) (BioMakor).
(c) Unseparated bone marrow (80 x 10⁶ cells per mouse, I.V.).
(d) Unseparated bone marrow (200 x 10⁶ cells per mouse, I.P.).
(e) Mononuclear bone marrow cells (15 x 10⁶ cells per mouse, I.V.).

The results are summarized in Table 1.

**Table 1:**

| Engraftment of Human T- and B-Lymphocytes Into Lethally Irradiated BALB/c Mice^{a} | | | | | |
|---|---|---|---|---|---|
| Weeks after transplant | Survival | Mice with human lymphocytes in peripheral blood | | | |
| Experiment 1 | | T-Cells | B-Cells | T+B-Cells | Human Ig^{b} |
| 7 weeks | 7/10 | 1/7 | N.T. | | |
| 10 weeks | 7/10 | 4/7 | N.T. | | |
| 16 weeks | 7/10 | 7/7 | N.T. | | |
| 22 weeks | 7/10 | 6/6 | 6/6 | 6/6 | 3/6 |

| Experiment 2 | | | | | |
|---|---|---|---|---|---|
| 7 weeks | 3/6 | 0/3 | N.T. | | |
| 18 weeks | 3/6 | 2/3 | 1/3 | 1/3 | 0/3 |

| Experiment 3 | | | | | |
|---|---|---|---|---|---|
| 9 weeks | 7/8 | 4/7 | 4/7 | 4/7 | 3/7 |

| Experiment 4 | | | | | |
|---|---|---|---|---|---|
| 11 weeks | 4/7 | 3/4 | 4/4 | 3/4 | 1/4 |

| Experiment 5^{c} | | | | | |
|---|---|---|---|---|---|
| 11 weeks | 7/8 | 6/7 | 5/7 | 5/7 | 2/7 |

| Experiment 6^{d} | | | | | |
|---|---|---|---|---|---|
| 11 weeks | 8/14 | 3/8 | 7/8 | 2/7 | 5/8 |

| Experiment 7^{e} | | | | | |
|---|---|---|---|---|---|
| 12 weeks | 7/7 | 5/7 | 7/7 | 5/7 | 1/7 |

As can be seen in Table 1, this approach has led to the development of human T and B cells in the peripheral blood of lethally irradiated normal BALB/c mice, which are H-2 identical with the SCID bone marrow donors. A total of 31 mice in 4 series of experiments received transplants of 10 x 10⁶ SBA⁻E⁻ human bone marrow cells by the intravenous route, followed one day later by an intravenously-administered transplant of 2 x 10⁶ T cell-depleted bone marrow cells from SCID mice. Of the 31 mice transplanted, 20 have survived for at least 3 months and up to more than 8 months. Mortality due to infection (10 mice out of 31) was observed mainly during the first 3 weeks post-transplant; one mouse died of infection during the fifth month post-transplant, following bleeding from the retro-orbital vein. Mice were periodically bled in order to assess engraftment of human lymphocytes in peripheral blood lymphocytes (PBL). Sixteen of the 20 survivors were found to be engrafted with human T cells, and/or human B cells. In 14/20 surviving mice, both T and B lymphocytes of human origin have been detected in significant numbers in the peripheral blood, beginning as early as 7 weeks and extending to up to 9 months post-transplant.

### Quantitative evaluation of T cell engraftment

A quantitative evaluation of T cell engraftment during the post-transplant period is best illustrated by the first series of mice, which have been observed for more than 6 months (Fig. 1). The appearance of human CD3⁺ T cells in peripheral blood of transplanted mice was first documented in one mouse (No. 3) 7 weeks post-transplant (Fig. 1, inset). In this mouse, 25% of PBL bound a monoclonal antibody specific for human CD3, compared to 66% of normal human PBL. In this assay, only 0.9% of PBL obtained from a mouse transplanted with SCID mouse marrow alone, were labeled with this reagent.

By the tenth week post-transplant, low but significant numbers of T cells were found in 4 out of 7 mice tested, ranging from 6.5% to 10.8% (an average of 7.9%). A marked increase in T cell numbers was found by the end of the fourth month post-transplant, at which time all 7 mice were found to be engrafted with human CD3⁺ T cells. In 6 out of 7 mice tested, the percentage of T cells present in peripheral blood was above 10%, ranging from 11.1% to 54.1% (an average of 27.1%). Similar numbers of human T cells were also recorded using monoclonal antibodies specific for human CD2 (data not shown).

Further phenotyping of the engrafted T cells with monoclonal antibodies specific for human CD8 and CD4 revealed abnormal or immature phenotypes, such as CD3⁺CD2⁺CD4⁻CD8⁻ lymphocytes commonly found in leukemia patients early after successful bone marrow transplantation with T cell-depleted bone marrow (Keever, C.A. et al. (1989) Blood 79, 1340). The percentage of these cells among the total human T cells in peripheral blood of engrafted mice 5 months post-transplant ranged from 49% to 84%. However, the remainder of the human T cells in peripheral blood were of mature phenotypes commonly found in normal peripheral blood, such as CD3⁺CD4⁺CD8⁻ and CD3⁺CD4⁻CD8⁺.

Thus, unlike the study in which a transient human T cell engraftment was detected only during the first month post-transplant (McCune, J.M. et al. (1988) Science 241, 1632), the experimental model of the invention demonstrates a slow but steady increase in human T cell numbers in peripheral blood of transplanted mice, beginning as early as 7 weeks post-transplant and reaching a plateau during the fourth and fifth months post-transplant (Fig. 1). These results are in accordance with the slow rate of T cell differentiation seen in human SCID patients and leukemia patients following transplantation of SBA⁻E⁻ bone marrow (Reisner, Y. et al. (1983) Blood 61, 341).

Examination by cytofluorimetry of thymocytes from transplanted mice 5 months (Fig. 2) or 6 months post-transplant (about 1-2 x 10⁶ viable cells could be collected after preparation of a thymocyte suspension in PBS) revealed a significant percentage of human cells bearing the phenotypes of normal human thymocytes. As in the normal human thymus, the major subpopulation of lymphocytes was found to bear the CD4⁺CD8⁺ phenotype. Smaller populations of CD4⁺CD8⁻ and CD4⁻CD8⁺ human lymphocytes were also detected (Fig. 2).

### Responsiveness of engrafted human T cells to mitogens

The responsiveness of engrafted human T cells to mitogens was evaluated by stimulation of splenocytes of human-mouse chimeras (from the third series) 5 months post- transplant, with a specific anti-human CD3 antibody, under conditions (OKT3/ATCC, Rockville, Maryland, USA, 1µg-2µg/well) used to test immune reconstitution in SCID or leukemia patients after bone marrow transplantation. This mitogen selectively stimulated human T cells and is not cross-reactive with murine cells. It is therefore more suitable than plant mitogens for evaluation of human-mouse chimeras.

Upon 7 days of culture with the mitogen, stimulation indexes were 3.1 and 2.2 in two chimeric mice tested, compared to 2.2 exhibited by human PBL and 0.8 by normal mouse splenocytes. However, the magnitude of the response of human-mouse chimeric splenocytes (the difference between the averages of thymidine incorporation obtained in the presence and in the absence of anti- OKT3) was only 10% (3178 cpm) and 7% (2440 cpm) respectively, of the normal human PBL response (32,707 cpm).

When spleen cells from the above chimeras were incubated with IL-2 containing medium and tested after 7 days of culture, by staining with anti-human CD3 antibody, human T cells were predominant (55%), in the surviving population, resembling the predominance of T cells observed when normal human PBL are cultured with these stimuli (83%) (Fig. 4), as opposed to results obtained with splenocytes from a control mouse transplanted with SCID bone marrow alone (1%). This shows that it is possible to clone human T cells from such human-mouse chimeras and thereafter, to test their specificity against the stimulating antigen.

### Testing for the presence of human B cells

The presence of human B cells in the peripheral blood of transplanted mice was initially tested in the first series of animals 5 months post-transplant. To distinguish human B cells in the human-mouse chimeras, we used directly-labeled monoclonal antibodies specific for the human B cell differentiation antigens CD19 and CD20. This test was performed twice, during the fifth and sixth months post-transplant. The proportions of human CD19⁺ and CD20⁺ cells in peripheral blood ranged from 4.5% to 16.3% for CD19, and from 3.5% to 33.0% for CD20 at 5 months post-transplant. Six months post-transplant, these values ranged from 9.0% to 30.4%, and from 2.1% to 20.6%, respectively. Typical staining of human B cells by anti-CD20 in peripheral blood and in the spleen of a transplanted mouse, as compared to staining of murine B cells in peripheral blood and in the spleen of a normal BALB/c mouse, is shown in Fig. 3.

The presence of B cells in PBL in the second and third series of bone marrow recipients was tested at relatively early time points post-transplant (Table 1). The earliest presence of human B cells was recorded 9 weeks post-transplant in 4 out of 7 mice from the third series. At this time, percentages of CD19⁺ human B cells in PBL of these mice ranged from 2.4% to 14.3%.

The function of engrafted human B lymphocytes has been assessed by testing levels of human immunoglobulins in the serum, and titrating human antibodies generated in response to KLH-DNP. Among mice transplanted with SBA⁻E⁻ human bone marrow (which is depleted of both T and B cells), 7/20 engrafted mice were positive for human Ig. In further testing of sera from 4 mice positive for human Ig, human IgM was detected in 2 animals and human IgG in 2 other mice. In subsequent experiments, mice were also transplanted with unmodified marrow (Table 1, see later) and 8/22 of these mice were positive for human Ig; human IgM was detected in two mice and human IgG in the other 6 animals.

Immunization of chimeric mice positive for human Ig (2 mice from the first series and 2 mice from the third series, 8 and 6 months post-transplant, respectively) with DNP-conjugated KLH, has induced both primary and secondary responses specific for DNP in 2/4 animals (Fig. 5). Following secondary immunization, specific human IgG as well as IgM could be detected in one of two mice. These results suggest that engrafted human B lymphocytes are able to function in a murine environment.

Murine PBL are not cross-reactive with the particular anti-human monoclonal antibodies used in our study (Figs. 1-3). Therefore, the cytofluorimetric analyses presented herein provide strong evidence that human T cells and B cells can grow and expand in transplanted normal BALB/c mice. To further clarify this issue, however, we confirmed the presence of human lymphocytes in the peripheral blood of transplanted mice by DNA analysis. As can be seen in Fig. 6, genomic DNA of the human (HLA-DQβ) genes was detected in the peripheral blood of mouse No. 1, whereas a test of normal PBL from an untreated mouse yielded completely negative results. In the same sample of PBL from mouse No. 1 (taken 6 months post-transplant), cytofluorimetry using anti-CD3 and anti- CD19 monoclonal antibodies revealed the presence of 60% human T and B lymphocytes, while staining with an anti-H2^{d} monoclonal antibody detected only 6.2% murine mononuclear cells.

Because of the presence of normal myeloid and erythroid precursors in the bone marrow of SCID mice, it was expected that when human SBA⁺E⁻ marrow cells were transplanted together with murine SCID bone marrow cells, engraftment of human myeloid cells would be negligible. However, although bone marrow preparations obtained from human-mouse lymphoid chimeras sacrificed at different times post-transplant have been predominantly of mouse origin when analyzed by staining with anti-H2^{d} antibody (specific for SCID and/or BALB/c strains of mice), cells reactive with antibodies directed against human myeloid differentiation antigens (anti-CD13 and anti-CD33) were repeatedly found in PBL (1-10%) (data not shown), as well as in occasional samples of bone marrow and spleen. The detected cells may include clonogenic progenitors with significant capacity for self-renewal and differentiation.

### Engraftment with unmodified bone marrow

Transplantation of human marrow mononuclear cell populations which had not been depleted of T cells was performed using the same transplant approach, but with larger doses (80 x 10⁶ cells/mouse) of unmodified marrow (to provide a relatively comparable number of progenitor cells). A rate of engraftment similar to that achieved with T cell-depleted bone marrow (Table 1) and without manifestations of graft-versus-host disease was observed.

In summary, the present invention provides "normal" mice having human B and T cells.

The foregoing written specification is considered by applicants to give sufficient information to enable a person skilled in the art to practice the invention. The examples presented herein of normal mice having B and T cells are intended as illustration of one embodiment of the invention, and the scope of the invention is not to be limited by the examples. Various modifications of the invention will become apparent to those skilled in the art from the foregoing description, and such modifications fall within the scope of the following claims.

## Claims

1. A chimeric rodent M4 having long-term stable xenogeneic hematopoietic cells, comprising a rodent M1, the hematopoietic cells of which have been substantially suppressed or destroyed and replaced by hematopoietic cells from at least two different sources, at least one of said sources being hematopoietic cells originating in a human M3, and at least a second of said sources being hematopoietic cells originating from a mammal M2, of a species other than that of M3, having a genetically-determined hematopoietic deficiency.

2. The chimeric rodent M4 according to claim 1 wherein said mammal M2 and said rodent M1 are of the same mammalian species.

3. The chimeric rodent M4 according to claim 2 wherein both mammal M2 and rodent M1 are mice.

4. The chimeric rodent M4 according to any one of claims 1 to 3 having human T lymphocytes.

5. The chimeric rodent M4 according to any one of claims 1 to 3 having human T lymphocytes and human B lymphocytes.

6. The chimeric rodent M4 according to any one of claims 1 to 3 having cells of the human erythroid cell lineage.

7. The chimeric rodent M4 according to any one of claims 1 to 3 having cells of the human myeloid lineage.

8. The chimeric mouse according to claim 3 wherein the hematopoietic cells of mouse M1 are replaced by human T cells and wherein said mammal M2 is a mouse with severe combined immunodeficiency (SCID).

9. The chimeric mouse according to claim 3 wherein the hematopoietic cells of rodent M1 are replaced by human T and B cells and wherein said mammal M2 is a mouse with severe combined immunodeficiency (SCID).

10. The chimeric mouse according to claim 9 wherein said rodent M1 is a BALB/c mouse.

11. A method for the production of a chimeric rodent M4 according to claim 1, which comprises:
(a) treating a rodent M1 so as to essentially destroy its immune system;
(b) transplanting said treated rodent M1 with hematopoietic cells originating in a human M3;
(c) additionally transplanting said treated rodent of step (b) with hematopoietic cells originating from a mammal M2, of a species other than that of M3, having a genetically-determined hematopoietic deficiency,
thereby obtaining said chimeric rodent M4 having hematopoietic cells derived from mammal donor M2 and further having one or more hematopoietic cell lineages from human donor M3, said hematopoietic cell lineages from human M3 corresponding to the cell lineages lacking from mammal M2 due to said genetically-determined hematopoietic deficiency.

12. The method according to claim 11 wherein the treatment of step (a) comprises chemotherapy, lethal irradiation or a combination of chemotherapy and lethal irradiation.

13. The method according to claim 11 or 12 wherein said human hematopoietic cells transplanted in step (b) are selected from the group consisting of normal bone marrow cells, mature blood cells, peripheral blood leukocytes, fetal liver cells, fetal thymus and fetal lymph nodes.

14. The method according to any one of claims 11 to 13 wherein said rodent M1 is a mouse, said mammal M2 is a mouse, and said human hematopoietic cells transplanted in step (b) are normal human bone marrow cells or any other suitable source of human pluripotent stem cells.

15. A method for the production of a chimeric mouse according to claim 9 having long term stable human B and T cells and hematopoietic cells derived from a SCID mouse, which comprises:
(a) lethally irradiating a recipient mouse such as to essentially destroy its immune system;
(b) transplanting said irradiated mouse with normal or T cell-depleted human bone marrow; and
(c) additionally transplanting said transplanted mouse of step (b) with T cell-depleted SCID mouse bone marrow cells,
thereby obtaining said chimeric mouse having long-term stable human B and T cells.

16. The method according to claim 15 wherein said chimeric mouse is a BALB/c mouse having long-term stable human B and T cells.

17. A method for the production of a human monoclonal antibody which comprises:
(a) immunizing a chimeric mouse according to claim 9 with an antigen;
(b) fusing spleen, thymus or lymph node cells of the immunized mouse with a suitable cultured fusion partner cell, thereby obtaining hybridoma cells;
(c) screening the supernatant of said hybridoma cells for the presence of the said human antibody;
(d) selecting and cloning the hybridoma producing said human antibody; and
(e) culturing the selected, cloned hybridoma in a suitable medium and recovering the antibody from the supernatant.

## Patentansprüche

1. Chimäres Nagetier M4, das langzeitstabile xenogene hämatopoietische Zellen hat, umfassend ein Nagetier M1, dessen hämatopoietische Zellen im wesentlichen supprimiert oder zerstört worden sind und durch hämatopoietische Zellen von mindestens zwei verschiedenen Spenderquellen ersetzt wurden, wobei mindestens eine der Spenderquellen hämatopoietische Zellen sind, die von einem Menschen M3 abstammen und mindestens eine zweite der Spenderquellen hämatopoietische Zellen sind, die von einem Säugetier M2 abstammen, das von einer anderen Art als jene von M3 ist, und das einen genetisch bestimmten hämatopoietischen Mangel hat.

2. Chimäres Nagetier M4 nach Anspruch 1, wobei das Säugetier M2 und das Nagetier M1 von derselben Säugetierart sind.

3. Chimäres Nagetier M4 nach Anspruch 2, wobei sowohl das Säugetier M2 als auch das Nagetier M1 Mäuse sind.

4. Chimäres Nagetier M4 nach einem der Ansprüche 1 bis 3, das menschliche T-Lymphocyten hat.

5. Chimäres Nagetier M4 nach einem der Ansprüche 1 bis 3, das menschliche T-Lymphocyten und menschliche B-Lymphocyten hat.

6. Chimäres Nagetier M4 nach einem der Ansprüche 1 bis 3, das Zellen der menschlichen erythrozytären Zellinie hat.

7. Chimäres Nagetier M4 nach einem der Ansprüche 1 bis 3, das Zellen der menschlichen myeloiden Linie hat.

8. Chimäre Maus nach Anspruch 3, wobei die hämatopoietischen Zellen der Maus M1 ersetzt sind durch menschliche T-Zellen, wobei das Säugetier M2 eine Maus mit schwerer kombinierter Immundefizienz (SCID) ist.

9. Chimäre Maus nach Anspruch 3, wobei die hämatopoietischen Zellen des Nagetiers M1 ersetzt sind durch menschliche T- und B-Zellen und wobei das Säugetier M2 eine Maus mit schwerer kombinierter Immundefizienz (SCID) ist.

10. Chimäre Maus nach Anspruch 9, wobei das Nagetier M1 eine BALB/c-Maus ist.

11. Verfahren zur Herstellung eines chimären Nagetiers M4 nach Anspruch 1, welches umfaßt:
(a) Behandlung eines Nagetiers M1, um sein Immunsystem im wesentlichen zu zerstören;
(b) Transplantation von hämatopoietischen Zellen, die vom menschlichen M3 stammen, in das behandelte Nagetier M1;
(c) zusätzliche Transplantation von hämatopoietischen Zellen, die von einem Säugetier M2 von einer anderen Art als jener von M3 stammen, und die einen genetisch bestimmten hämatopoietischen Mangel haben, in das behandelte Nagetier von Schritt (b),
wodurch das Nagetier M4 erhalten wird, das hämatopoietische Zellen hat, die von einem Säugetier-Donor M2 stammen, und weiterhin eine oder mehrere hämatopoietische Zellinien von einem menschlichen Donor M3 hat, wobei die hämatopoietischen Zellinien von einem Mensch M3 den Zellinien entsprechen, die dem Säugetier M2 durch den genetisch bestimmten hämatopoietischen Mangel fehlen.

12. Verfahren nach Anspruch 11, wobei die Behandlung in Schritt (a) eine Chemotherapie, tödliche Bestrahlung oder eine Kombination von Chemotherapie und tödlicher Bestrahlung umfaßt.

13. Verfahren nach Anspruch 11 oder 12, wobei die menschlichen hämatopoietischen Zellen, die im Schritt (b) transplantiert werden, ausgewählt sind aus normalen Knochenmarkszellen, reifen Blutzellen, peripheren Blutleukocyten, fötalen Leberzellen, fötalem Thymus oder fötalen Lymphknoten.

14. Verfahren nach einem der Ansprüche 11 bis 13, wobei das Nagetier M1 eine Maus ist, das Säugetier M2 eine Maus ist und die menschlichen hämatopoietischen Zellen, die im Schritt (b) transplantiert werden, normale menschliche Knochenmarkszellen oder Zellen einer anderen geeigneten Quelle von menschlichen pluripotenten Stammzellen sind.

15. Verfahren zur Herstellung einer chimären Maus nach Anspruch 9, die langzeitstabile menschliche B- und T-Zellen und hämatopoietische Zellen, die von einer SCID-Maus stammen, hat, umfassend:
(a) tödliche Bestrahlung einer Empfängermaus, so daß deren Immunsystem im wesentlichen zerstört wird;
(b) Transformation von normalem oder T-Zell-entzogenem menschlichen Knochenmark in die bestrahlte Maus; und
(c) zusätzliche Transplantation von T-Zell-entzogenen SCID-Maus-Knochenmarkzellen in die transplantierte Maus von Schritt (b),
wodurch die chimäre Maus erhalten wird, die langzeitstabile menschlichen B- und T-Zellen hat.

16. Verfahren nach Anspruch 15, wobei die chimäre Maus eine BALB/c-Maus ist, die langzeitstabile menschliche B- und T-Zellen hat.

17. Verfahren zur Herstellung eines menschlichen monoclonalen Antikörpers, umfassend:
(a) Immunisieren einer chimären Maus nach Anspruch 9 mit einem Antigen;
(b) Fusionieren von Milz-, Thymus- oder Lymphknotenzellen der immunisierten Maus mit geeigneten gezüchteten Fusionspartnerzellen, wobei Hybridomzellen erhalten werden;
(c) Absuchen der Überstände der Hybridomzellen auf die Anwesenheit des menschlichen Antikörpers;
(d) Auswahl und Clonierung des Hybridoms, das den menschlichen Antikörper produziert; und
(e) Züchten des ausgewählten und clonierten Hybridoms in einem geeigneten Medium und Gewinnung des Antikörpers aus dem Überstand.

## Revendications

1. Rongeur chimère M4 ayant des cellules hématopoïétiques xénogéniques stables à long terme, comprenant un rongeur M1, dont les cellules hématopoïétiques ont été sensiblement supprimées ou détruites et remplacées par des cellules hématopoïétiques provenant d'au moins deux sources différentes, au moins l'une desdites sources étant des cellules hématopoïétiques provenant d'un être humain M3, et au moins une deuxième desdites sources étant des cellules hématopoïétiques provenant d'un mammifère M2, ou d'une espèce autre que celle de M3, ayant une déficience hématopoïétique génétiquement déterminée.

2. Rongeur chimère M4 selon la revendication 1, dans lequel ledit mammifère M2 et ledit rongeur M1 sont de la même espèce mammifère.

3. Rongeur chimère M4 selon la revendication 2, dans lequel tant le mammifère M2 que le rongeur M1 sont des souris.

4. Rongeur chimère M4 selon l'une quelconque des revendications 1 à 3, ayant des lymphocytes T humains.

5. Rongeur chimère M4 selon l'une quelconque des revendications 1 à 3, ayant des lymphocytes T humains et des lymphocytes B humains.

6. Rongeur chimère M4 selon l'une quelconque des revendications 1 à 3, ayant des cellules de la lignée de cellules érythrocytaires humaine.

7. Rongeur chimère M4 selon l'une quelconque des revendications 1 à 3, ayant des cellules de la lignée myéloïde humaine.

8. Souris chimère selon la revendication 3, dans laquelle les cellules hématopoïétiques de souris M1 sont remplacées par des cellules T humaines et dans laquelle ledit mammifère M2 est une souris ayant un déficit immunitaire combiné sévère (DICS).

9. Souris chimère selon la revendication 3, dans laquelle les cellules hématopoïétiques de rongeur M1 sont remplacées par des cellules T et B et dans laquelle ledit mammifère M2 est une souris ayant un déficit immunitaire combiné sévère (DICS).

10. Souris chimère selon la revendication 9, dans laquelle ledit rongeur M1 est une souris BALB/c.

11. Procédé pour la production d'un rongeur chimère M4 selon la revendication 1, qui consiste à :
(a) traiter un rongeur M1 de façon à détruire sensiblement son système immunitaire ;
(b) transplanter dans ledit rongeur M1 traité des cellules hématopoïétiques provenant d'un humain M3 ;
(c) transplanter de plus dans ledit rongeur traité de l'étape (b) des cellules hématopoïétiques provenant d'un mammifère M2, d'une espèce autre que celle de M3, ayant une déficience hématopoïétique génétiquement déterminée ;
de façon à obtenir ainsi ledit rongeur chimère M4 ayant des cellules hématopoïétiques dérivant du donneur mammifère M2 et ayant en outre une ou plusieurs lignées de cellules hématopoïétiques provenant du donneur humain M3, lesdites lignées de cellules hématopoïétiques provenant d'être humain M3 correspondant aux lignées de cellules manquantes du mammifère M2 du fait d'une déficience hématopoïétique génétiquement déterminée.

12. Procédé selon la revendication 11, dans lequel le traitement de l'étape (a) comprend une chimiothérapie, une irradiation létale ou une combinaison de chimiothérapie et d'irradiation létale.

13. Procédé selon la revendication 11 ou 12, dans lequel lesdites cellules hématopoïétiques humaines transplantées dans l'étape (b) sont choisies dans le groupe constitué par les cellules de moelle osseuse normales, les globules sanguins matures, les leucocytes de sang périphérique, les cellules de foie foetal, le thymus foetal et les ganglions lymphatiques foetaux.

14. Procédé selon l'une quelconque des revendications 11 à 13, dans lequel ledit rongeur M1 est une souris, ledit mammifère M2 est une souris, et lesdites cellules hématopoïétiques humaines transplantées dans l'étape (b) sont des cellules de moelle osseuse humaine normale ou n'importe quelle autre source convenable de cellules souches pluripotentes humaines.

15. Procédé pour la production d'une souris chimère selon la revendication 9 ayant des cellules B et T humaines stables à long terme et des cellules hématopoïétiques dérivant d'une souris DICS, qui consiste à :
(a) irradier à dose létale une souris receveuse de façon à détruire essentiellement son système immunitaire ;
(b) transplanter dans ladite souris irradiée de la moelle osseuse humaine normale ou appauvrie en cellules T ; et
(c) transplanter de plus à ladite souris transplantée de l'étape (b) des cellules de moelle osseuse de souris DICS appauvrie en cellules T,
de façon à obtenir ainsi ladite souris chimère ayant des cellules B et T humaines stables à long terme.

16. Procédé selon la revendication 15, dans lequel ladite souris chimère est une souris BALB/c ayant des cellules B et T humaines stables à long terme.

17. Procédé pour la production d'un anticorps monoclonal humain, qui consiste à :
(a) immuniser une souris chimère selon la revendication 9 avec un antigène ;
(b) fusionner des cellules de rate, de thymus ou de ganglions lymphatiques de la souris immunisée avec une cellule partenaire de fusion cultivée convenable, de façon à obtenir ainsi des cellules d'hybridome ;
(c) cribler la présence dudit anticorps humain dans le surnageant desdites cellules d'hybridome ;
(d) sélectionner et cloner l'hybridome produisant ledit anticorps humain ; et
(e) cultiver l'hybridome cloné sélectionné, dans un milieu convenable, et récupérer l'anticorps à partir du surnageant.
